# EUROPEAN PATENT APPLICATION

(11) **EP 1 978 497 A1**
(43) Date of publication of application: **08.10.2008**
(21) Application number: 07105753.3
(22) Date of filing: 05.04.2007
(51) Int. Cl.: G09F 3/00, A63B 24/00, G06F 15/00

(54) **Method and system for providing an exercise goal**

(71) Applicant: Koninklijke Philips Electronics N.V., 5621 BA Eindhoven (NL)
(72) Inventor: The designation of the inventor has not yet been filed
(74) Representative: Schoenmaker, Maarten

(57) **Abstract**

The present invention provides a method and a corresponding system for providing an exercise goal, comprising the steps of - providing a database, comprising a reference success rate, and a reference exercise goal that is coupled to the reference success rate, wherein the reference success rate is the percentage of a predetermined number of people who were successful in achieving the reference exercise goal; - providing personal data of the user; - providing a selection criterion; and - determining the exercise goal on the basis of the selection criterion, the personal data, and the coupled reference success rate and the reference exercise goal. A user may either input a desired exercise goal, or input a desired success rate. The method will output the corresponding reference success rate or exercise goal.

## Description

### FIELD OF THE INVENTION

The present invention relates to a system and a method for setting an exercise goal. Applications of the method and system include for instance an exercise routine, preventive or personal healthcare, motivation of consumers, a decision support system, and/or a weight loss program.

### BACKGROUND OF THE INVENTION

US-2006/0040793-A1 provides a system and a method for providing visual feedback to a user of an exercise machine for gauging fitness progress of the user. The system provides the user with a virtual competition in which the user competes against virtual competitors based on his past performances or those of other users. For an individual competing against his own past performances, the system may raise the level of performance required to win the virtual competition, and may also lower the level of performance required if the user is not performing well on a particular day. The system attempts to keep the user engaged and motivated to achieve desired fitness goals by providing real-time performance data and historical performance data displayed in a graphical manner coupled with the entertainment and excitement of competition and social interaction.

The above prior art system assists the user in complying with an exercise program and the progress towards a desired fitness level. The user sets the desired fitness level himself, possibly with the help of for instance a trainer, dietician or physician. However, the user tends to overestimate his abilities and to set unrealistic, unachievable goals. Unrealistic goals will result in loss of motivation and engagement.

### OBJECT OF THE INVENTION

The present invention therefore aims to provide a system and a method for setting a more realistic goal.

### SUMMARY OF THE INVENTION

The present invention therefore provides a method for providing a user with an exercise goal, comprising the steps of:
- providing a database, comprising a reference success rate, and a reference exercise goal that is coupled to the reference success rate, wherein the reference success rate is the percentage of a predetermined number of people who were successful in achieving the reference exercise goal;
- providing personal data of the user;
- providing a selection criterion; and
- determining the exercise goal on the basis of the selection criterion, the personal data, and the coupled reference success rate and the reference exercise goal.

The method of the present invention uses historical data of people who have been successful in achieving the pre-set goal of their exercise to evaluate the goal of the user. The method uses a database, wherein reference data of said people are coupled to their exercise goal. The way of evaluating the goal may differ, see the embodiments of the subclaims below. Also, the contents of the reference data are provided in the subclaims.

In an embodiment, the database comprises multiple reference success rates and multiple reference exercise goals that are coupled to a corresponding reference success rate of the multiple reference success rates.

By increasing the amount of reference data, i.e. by including data of multiple people who have been successful in achieving the pre-set goal of their exercise, the accuracy of the advice also increases.

In a further embodiment, the method comprises the steps of: - inputting a desired exercise goal; and - outputting a reference success rate that corresponds to the desired exercise goal.

In another embodiment, the method comprises the steps of: - inputting a desired success rate; and - outputting an exercise goal that corresponds to the desired success rate.

The above indicates that the method provides the user with the ability to either input a desired exercise goal, or a desired success rate. The method will subsequently evaluate the input, and provide the user with the results of the evaluation. Possibly, the results may include at least part of the reference data included in the database.

Optionally, the personal data comprise one or more of: - initial weight, target weight, age, gender, waist-to-hip ratio, daily calorie intake, food composition, daily or weekly time budget for exercise, average running speed over a predetermined distance, and time to achieve the exercise goal. The database may further comprise reference data of the people who were successful in achieving the reference exercise goal, comprising one or more of: - initial weight, target weight, age, gender, waist-to-hip ratio, daily calorie intake, food composition, perseverance, endurance, daily or weekly time budget for exercise, average running speed over a predetermined distance, and time to achieve the exercise goal.

In an embodiment, the step of determining the exercise goal comprises the steps of:
- inputting the personal data of the user;
- pattern matching the personal data to the reference data;
- determining the number of people comprised in the reference data having a matching data pattern, wherein the selection criterion is user to determine if the separate data match;
- using the determined number of people to determine a reference success rate and/or a reference exercise goal; and
- outputting an exercise goal that corresponds to the determined reference success rate and/or the reference exercise goal.

In this embodiment, the user inputs personal data. The method subsequently evaluates the input, determines a success rate of the input and provides a suitable exercise goal. The embodiment may use fuzzy logic to evaluate the input. This embodiment stresses the flexibility of the method and system of the invention.

In another embodiment, the method comprises the steps of:
- inputting the personal data of the user;
- inputting either one of desired success rate or desired exercise goal;
- transferring the inputted desired success rate or desired exercise goal to the database and comparing it to the reference success rate and a corresponding exercise goal;
- obtaining the reference success rate or reference exercise goal that corresponds to the inputted desired success rate or desired exercise goal.

The user inputs personal data, as well as a desired success rate or a desired exercise goal. The method subsequently evaluates the input, and provides a suitable exercise goal within the restrictions comprised in the input. The embodiment may use statistical rules to evaluate the input. This embodiment shows the flexibility of the method and system of the invention.

In an embodiment, the reference success rates and the reference exercise goals comprised in the database are arranged as rules, wherein the rules are statistically determined. Using the pre-set criterion and the statistical rules will result in a more realistic exercise goal than the user would set himself.

Optionally, iteration is used to provide a success rate and/or an exercise goal in between two adjacent values comprised in the rules in the database.

According to another aspect, the present invention provides a system for providing a user with an exercise goal, comprising:
- a database, comprising a reference success rate, and a reference exercise goal that is coupled to the reference success rate, wherein the reference success rate is the percentage of a predetermined number of people who were successful in achieving the reference exercise goal;
- input means for inputting personal data of the user, a desired exercise goal and/or a desired success rate;
- output means for outputting the reference success rate that corresponds to the desired exercise goal, and/or for outputting the exercise goal that corresponds to the desired success rate; and
- processing means, which are coupled to the database, the input means and the output means, for determining the exercise goal on the basis of a selection criterion, the personal data, and the reference success rate and the reference exercise goal.

The system of the present invention includes a database comprising historical data of people who have been successful in achieving the pre-set goal of their exercise. The system uses the database to evaluate the exercise goal of the user. In the database, reference data of said people are coupled to their exercise goal.

To increase the accuracy of the advice, the database preferably comprises multiple coupled reference success rates and corresponding exercise goals.

In an embodiment, the database further comprises: - reference data and an exercise goal of a predetermined number of people who were successful in achieving their exercise goal. Including reference data of the people, besides their exercise goal and reference success rate, increases the applicability of the system. Fuzzy logic may be applied to evaluate the input and to provide the user with a suitable exercise goal.

### BRIEF DESCRIPTION OF THE DRAWINGS

Fig. 1 shows a schematic depiction of an embodiment of the system according to the present invention;
Fig. 2 shows an example of a rule scheme of coupled reference data and reference success rates wherein the data concern weight loss;
Fig. 3 shows an example of a rule scheme of coupled reference data and reference success rates wherein the data concern average distance of running per week; and
Fig. 4 shows a rule scheme of coupled reference data and reference success rates wherein the data concern maximum calorie intake per day.

### DETAILED DESCRIPTION OF EXAMPLES

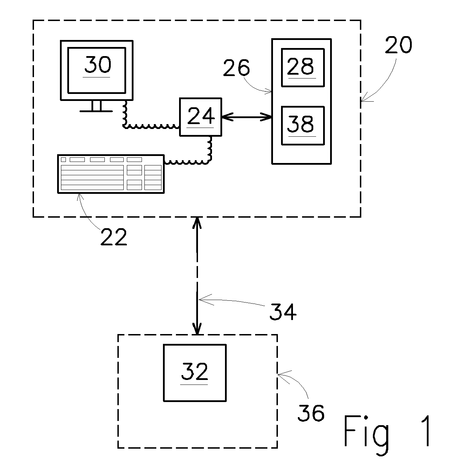

In an embodiment, a system 10 according to the present invention comprises a user device 20. The user device comprises input means 22 for inputting data. The input means comprise in the embodiment of Fig. 1 a keyboard. The input means may further include for instance a microphone or a data connection with an external electronic device. The input means are coupled to a processor 24. The processor 24 is coupled to a memory 26, comprising a database 28. Output means comprising a screen 30 are also coupled to the processor.

The user device is connected to an update means 32 via data connection 34. The data connection may include any suitable electronic connection means, including data cables, wireless data transfer systems, server systems and/or routers. The update device may comprise any suitable update means. The update device may be included in the Internet 36, for instance as an update server system at a remote location. The remote location is for instance the location of a service provider, or the location of the producer of the system of the present invention.

The system 10 is adapted to advice a user based on information comprised in the database 28. To provide the most up to date information, the information is preferably updated at predetermined times via the data connection 34 and the update means 32.

In a preferred embodiment, the update device is coupled to a number of other devices (not shown), comparable to device 20. The update device 32 comprises a database that may be updated by including all the exercise data of users that are successful in achieving their pre-set goals. Such assembly of devices and the update device 32 may function as a sort of community, for instance on the Internet.

In an embodiment, the information of the database 28 comprises historical reference data of a plurality of people who were successful in achieving the goal of their exercise program. The reference data comprise at least the initial status of the plurality of people, and their exercise goal.

Data representing initial status for instance include initial weight, target weight, age, gender, waist-to-hip ratio, daily calorie intake, food composition (i.e. carbohydrates, protein, fat), perseverance, endurance, daily or weekly time budget for exercise, average running speed over a predetermined distance, and/or the time that is available to achieve the exercise goal. The predetermined distance is for instance 500 m, 1 km, 2 km, 3 km, 5 km, and 10 km.

The exercise goal may include a desired target weight, waist-to-hip ratio, daily calorie intake, food composition, daily or weekly time budget for exercise, and/or average running speed over a predetermined distance. Other such goals related to the physical condition of the user are imaginable.

The database 28 may be stored in the memory 26 of the device 20. The device 20 may be any suitable electronic device, for instance a personal computer, a mobile telephone or a Personal Digital Assistant (PDA). The examples of the mobile telephone and the PDA indicate that the device 20 may be portable, so the user can take the device with him on journeys, to the gym et cetera.

The user of the device 20 inputs his or her personal data 38 using the keyboard 22. The personal data represent the initial status of the user, and comprise one or more of the data also comprised in the reference data. The personal data 38 may be stored in the memory 26 for repeated use.

In an embodiment, the processor compares the personal data to the reference data. The comparison may be displayed on the screen 30, to enable the user to compare the personal and the reference data himself.

Otherwise, the processor may compare the personal and the reference data, and/or the goals set. When comparing both data, the processor checks how many people having similar data and/or a similar exercise goal, or goals, are included in the database. Similar here indicates the presence of a pre-set criterion, for instance a predetermined bandwidth. If the exercise goal and/or the success rate within said bandwidth, the data are considered to be similar. The bandwidth may be expressed as a percentage, preferably having a success rate within the range of 80% to 100%. Otherwise, the bandwidth may indicate that for instance 80% to 100% of the data should match, allowing a 0 to 20% difference in personal data between people in the reference database and the user. For a more realistic advice, the bandwidth is smaller, within the range of 90% to 100%.

The reference data and the personal data of the user are compared to determine the number of people included in the reference data having similar characteristics, and/or a similar goal. Said number of people in the reference data indicates how realistic the pre-set goal of the user is. The more people having similar data were successful, the more realistic the pre-set goal is.

Said number of people may for instance be expressed as a percentage of the total number of people in the database. If the total number of people in the database is 1000, a pre-set goal may for instance be assumed realistic if more than 10%, i.e. if more than 100 people included in the database were previously successful in achieving the same goal. The more similar cases are represented in the database, the more realistic the goal is.

The number of people comprised in the database may vary. However, the accuracy of the advice will improve if the database comprises an increasing amount of reference data, i.e. with an increasing number of people. The total number of people for instance may be 100, or more. Preferably, the total number of people is more than 10000. The latter case allows to include unusual cases in the database. Thus, the ability of the system and method to deal with unusual cases also improves.

In another embodiment, the database 28 includes statistical data. The statistical data may be obtained from various sources, such as scientific studies, weight loss studies, and/or historical data from sports academies.

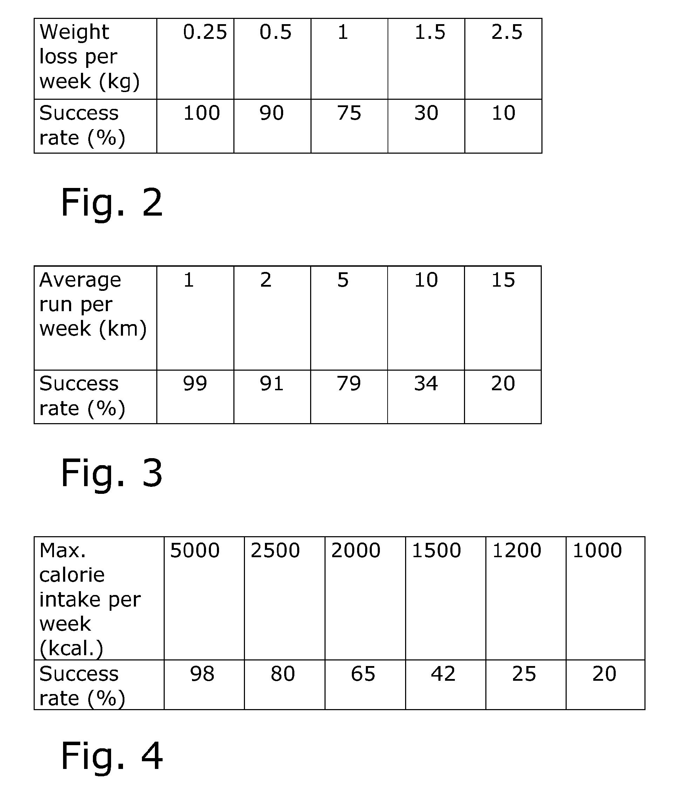

Figs. 2-4 show examples of the statistical data. Fig. 2 shows the average weight loss per week and a corresponding reference success rate of a reference group of people. Likewise, Fig. 3 shows the average distance of running per week and a corresponding reference success rate of a reference group of people. Fig. 4 shows maximum calorie intake per week and a corresponding reference success rate of a reference group of people.

The user can use the system 10 in at least two different ways. In a first case, the user inputs a desired exercise goal. The system subsequently determines the corresponding reference success rate from the stored statistical rules, as shown in Figs. 2-4. The system 10 displays the corresponding reference success rate, and/or the statistical data as represented in one or more of Figs. 2-4, on the screen 30.

Referring to Fig. 2, the user may for instance desire to lose weight. As the user is highly motivated, he or she desires to lose two kilos per week. The system subsequently provides the user with an iterated reference success rate of about 20%. As background information, the whole list shown in Fig. 2 may be provided, to enable the user to adapt the goal accordingly.

In a more elaborate embodiment, the user also inputs his personal data as mentioned above. The processor may use said personal data to determine a criterion. For instance, a pre-set criterion for success may be 90%. For instance, as default the system may advice an exercise goal that 90% of all people are expected to achieve. If the user is highly motivated, the reference success rate could be lowered proportionally. If the user is more motivated than, say, 75% of a reference group of people, the system could advice an exercise goal having a corresponding reference success rate of 65%. Likewise, the system 20 may adapt the criterion if the user has a relatively high body weight, which could for instance proportionally reduce the endurance and/or increase the daily calorie intake of the user.

In a second case, the user inputs a desired success rate for certain exercise data. Again, the user is highly motivated and inputs a desired success rate of 75%. The system subsequently provides the user with a corresponding exercise goal.

Referring to Fig. 4, the user may for instance desire to lower his or her calorie intake per week. As the user is a chef, the inputted success rate is 85%. The system subsequently provides the user with an iterated reference maximum calorie intake of about 3000 kcal per week. As background information, the whole list shown in Fig. 4 may be provided, to enable the user to adapt the goal accordingly.

The method of the present invention thus provides at least four different embodiments. Firstly, the user may input either a desired goal, or a desired success rate. Secondly, the method may use either compare the user data and reference data using a pattern-matching algorithm, or use statistical data to determine the corresponding exercise advice.

The processor 24 may include programs to adapt stored reference data in the database to unusual cases. Examples of such programs include fuzzy logic. Also, the programs may determine a suitable success rate for the provided user data. For instance, as motivation is deemed highly important, the expected success rate could be pre-set at 90%. Dependent on the user data, the pre-set success rate may be adapted, depending on the user data and/or the above-mentioned scientific studies et cetera. For example, the system may amend the pre-set criterion depending on initial weight, medical history, age, gender, and/or fitness of the user.

As mentioned before, the method and system according to the present invention assist a user in setting a (more) realistic goal for an exercise. The exercise includes for instance an exercise routine, a fitness training in a gym, preventive or personal healthcare, a diet and/or a weight loss program. The fitness training comprises for instance one or more of weight lifting, running, steps, aerobics, and/or rowing. The fitness training may, and in general will be combined with any other exercise, such as a diet. The diet comprises advice on and/or monitoring of eating habits and patterns. The diet thus is concerned with daily calorie intake, for instance at breakfast, lunch and dinner, and/or with food composition. The food composition concerns the percentage of fat, protein and carbohydrates of the food that the user consumes per day.

More in general, the method and system of the present invention may be comprised in a decision support system, or in a system for the motivation of consumers. The latter is for instance available in shops, and functions as a marketing and/or sales tool. The sales tool comprising the system of the present invention assists in improving sales and increases customer satisfaction, as customers are sold items that suit their personal data.

The present invention is not limited to the embodiments described above. Many modifications therein can be envisaged within the scope of the appended claims. Likewise, features of separate embodiments may be combined within the scope of the claims.

## Claims

1. A method for providing a user with an exercise goal, comprising the steps of:
- providing a database, comprising a reference success rate, and a reference exercise goal that is coupled to the reference success rate, wherein the reference success rate is the percentage of a predetermined number of people who were successful in achieving the reference exercise goal;
- providing personal data of the user;
- providing a selection criterion; and
- determining the exercise goal on the basis of the selection criterion, the personal data, and the coupled reference success rate and the reference exercise goal.

2. The method of claim 1, wherein the database comprises multiple reference success rates and multiple reference exercise goals that are coupled to a corresponding reference success rate of the multiple reference success rates.

3. The method of claim 1 or 2, comprising the steps of:
- inputting a desired exercise goal; and
- outputting a reference success rate that corresponds to the desired exercise goal.

4. The method of claim 1 or 2, comprising the steps of:
- inputting a desired success rate; and
- outputting an exercise goal that corresponds to the desired success rate.

5. The method of any of claims 1-4, wherein the personal data comprise one or more of:
- initial weight, target weight, age, gender, waist-to-hip ratio, daily calorie intake, food composition, motivation level, initial calorie expenditure, daily or weekly time budget for exercise, average running speed over a predetermined distance, and time to achieve the exercise goal

6. The method of any of claims 1-5, wherein the exercise goal is a goal for an exercise comprising one or more of:
- an exercise routine,
- a fitness training, comprising one or more of weight lifting, running, cycling, aerobics, and/or rowing;
- preventive or personal healthcare,
- a diet, comprising eating habits and patterns, daily calorie intake, and/or food composition concerning the percentage of fat, protein and carbohydrates of the food that the user consumes per day; and/or
- a weight loss program.

7. The method of any of claims 1-6, wherein the database further comprises reference data of the people who were successful in achieving the reference exercise goal, comprising one or more of:
- initial weight, target weight, age, gender, waist-to-hip ratio, daily calorie intake, food composition, motivation level, initial calorie expenditure, perseverance, endurance, daily or weekly time budget for exercise, average running speed over a predetermined distance, and time to achieve the exercise goal.

8. The method of any of claims 1-7, wherein the step of determining the exercise goal comprises the steps of:
- inputting the personal data of the user;
- pattern matching the personal data to the reference data;
- determining the number of people comprised in the reference data having a matching data pattern, wherein the selection criterion is user to determine if the separate data match;
- using the determined number of people to determine a reference success rate and/or a reference exercise goal; and
- outputting an exercise goal that corresponds to the determined reference success rate and/or the reference exercise goal.

9. The method of any of claims 1-7, comprising the steps of:
- inputting the personal data of the user;
- inputting either one of desired success rate or desired exercise goal;
- transferring the inputted desired success rate or desired exercise goal to the database and comparing it to the reference success rate and a corresponding exercise goal;
- obtaining the reference success rate or reference exercise goal that corresponds to the inputted desired success rate or desired exercise goal.

10. The method of claim 9, wherein the reference success rates and the reference exercise goals comprised in the database are arranged as rules, wherein the rules are statistically determined.

11. The method of claim 10, wherein iteration is used to provide a success rate and/or an exercise goal in between two adjacent values comprised in the rules in the database.

12. The method of any of claims 1-10, wherein the selection criterion is a desired success rate, represented as a percentage.

13. The method of claim 12, wherein the percentage is within the range of 80-100%, preferably about 90%.

14. System for providing a user with an exercise goal, comprising:
- a database, comprising a reference success rate, and a reference exercise goal that is coupled to the reference success rate, wherein the reference success rate is the percentage of a predetermined number of people who were successful in achieving the reference exercise goal;
- input means for inputting personal data of the user, a desired exercise goal and/or a desired success rate;
- output means for outputting the reference success rate that corresponds to the desired exercise goal, and/or for outputting the exercise goal that corresponds to the desired success rate; and
- processing means, which are coupled to the database, the input means and the output means, for determining the exercise goal on the basis of a selection criterion, the personal data, and the reference success rate and the reference exercise goal.

15. The system of claim 14, wherein the database comprises multiple coupled reference success rates and corresponding exercise goals.

16. The system of claim 14 or 15, wherein the database further comprises:
- reference data and an exercise goal of a predetermined number of people who were successful in achieving their exercise goal.

17. The system of claim 16, wherein the reference data comprise one or more of:
- initial weight, target weight, age, gender, waist-to-hip ratio, daily calorie intake, food composition, motivation level, initial calorie expenditure, daily or weekly time budget for exercise, average running speed over a predetermined distance, and time to achieve the exercise goal.
